# EUROPEAN PATENT SPECIFICATION

(11) **EP 0 931 157 B1**
(45) Date of publication and mention of the grant of the patent: **27.08.2008**
(21) Application number: 97909438.0
(22) Date of filing: 17.10.1997
(51) Int. Cl.: C12N 15/86, C12N 5/10, C12N 7/00, A61K 48/00, C07K 14/16

(54) **RETROVIRAL VECTORS**
RETROVIRALE VEKTOREN
VECTEURS RETROVIRAUX

(30) Priority: 17.10.1996 GB 9621679
(43) Date of publication of application: 28.07.1999
(73) Proprietor: Oxford Biomedica (UK) Limited, Oxford 0X4 4GA (GB)
(72) Inventor: KINGSMAN, Susan Mary, Islip, Oxon OX5 2SF (GB); KINGSMAN, Alan John, Islip, Oxon OX5 2SF (GB)
(74) Representative: Harding, Charles Thomas
(86) International application number: PCT/GB1997/002859
(87) International publication number: WO 1998/017817

(56) References cited:
- WO-A-92/21750
- WO-A-95/30755
- US-A- 5 306 631
- HOPE, T.J. ET AL.: "Steroid-receptor fusion of the human immunodeficiency virus type 1 Rev transactivator: mapping cryptic functions of the arginine-rich motif" PROCEEDINGS OF THE NATIONAL ACADEMY OF SCIENCE OF THE USA, vol. 87, October 1990, pages 7787-7791, XP002056927
- MCLACHLIN J R ET AL: "RETROVIRAL-MEDIATED GENE TRANSFER" PROGRESS IN NUCLEIC ACID RESEARCH AND MOLECULAR BIOLOGY, vol. 38, 1990, pages 91-135, XP002030826

## Description

This invention relates to retroviral vector particles and to DNA constructs encoding RNA genomes for retroviral vectors. In particular it relates to retroviral vectors for gene therapy for treatment or prevention of retrovirus infections, such as HIV.

Therapeutic molecules for use in HIV gene therapy include ribozymes, trans-dominant proteins, scFv molecules, antisense constructs and TAR and RRE decoys (reviewed in Yu *et al.* 1994). These molecules are envisaged to act both as therapy against already infected cells and as protective 'intracellular immunisation' (Baltimore, 1988) in uninfected cells. In addition, the use of toxins (suicide genes) or immunological markers has also been proposed as a means of killing infected cells and so reducing the viral load in the patient.

For many of these molecules, it is desirable that expression can be regulated. This is clearly the case for suicide genes, but the constitutive expression of a therapeutic protein may also be undesirable in that it may cause some cellular toxicity or lead to a host immunological response. Furthermore, toxic side effects are also possible from the expression of RNA molecules in cells, including the inappropriate induction of interferon responses. In the case of HIV, the viral LTR promoter itself has the characteristic of being an inducible promoter, directing low basal levels of transcription in the absence of the virally encoded Tat transactivator protein (Arya *et al.* 1985). Tat-inducibility is a property of sequences in the U3 region of the LTR and the TAR sequence in the R region (Berkhout and Jeang, 1992). Allowing the HIV LTR to direct expression of a therapeutic gene will therefore limit its expression to those cells infected by HIV. Several groups have already started exploring ways of using this property of the LTR and have demonstrated inducible expression of genes upon HIV infection (Caruso *et al.* 1992, Brady *et al.* 1994). Recently we have developed a novel vector system, the TIN system, that has clear advantages for HIV gene therapy (PCT/GB96/01230). This system comprises novel MLV based Tat-inducible (TIN) vectors, designed for the 5' positioning of a Tat induced therapeutic gene (TITG), and incorporating features to allow the efficient packaging, reverse transcription and integration of the vector genome by the MLV machinery. To facilitate the production of high levels of vector genomes in the producer cells (standard MLV packaging lines or our transient transfection system (Soneoka *et al.* 1995)), the packagable vector transcript is preferably driven from the CMV promoter rather than the natural vector MLV LTR, A clear advantage of this system is the achievement of HIV control of gene expression from a simple transcription unit which can be transduced by the standard MLV based vector technology. The TIN vector system was proposed to have additional advantages in that the low basal levels of transcription from the HIV LTR promoter would reduce problems of interference with any downstream constitutive promoters, facilitating stable expression of any constitutive gene from the vector in the transduced cells.

Where the therapeutic gene is a non natural protein there always exists the possibility of immune recognition and destruction of the cell expressing this protein. This is clearly undesirable if the aim is to create a reservoir of HIV resistant cells in the patient. It is therefore important to have basal levels of expression as close to zero as possible. The data using TIN vectors that we have previously described clearly demonstrate very low basal levels of expression but the levels were still detectable. Furthermore it is known that in some target cells additional cellular factors may be present that enhance the basal transcription from the HIV promoter in the absence of Tat. Indeed some transcription from the HIV LTR must occur to produce Tat itself. In the T cell population exposure to cytokines, antigens and various stress factors such as hypoxia or UV irradiation are all known to stimulate transcription from the HIV LTR (Valerie *et al 1988;* Nabel & Baltimore 1987). Given that these types of stimuli are characteristic features of immune responses there therefore exists the possibility that basal expression from TIN vectors could be elevated under certain conditions within an HIV infected patient. It is therefore desirable to provide additional controls on the basal levels of expression.

There is an additional regulatory circuit in HIV gene expression which involves the virally encoded Rev protein and its target RNA sequence the Rev response element (RRE) (reviewed by Cullen 1995 and explored by Naldini *et al 1996).* HIV has two introns in its genome and regulates splicing to generate unspliced RNA genomes and two classes of sub-genomic mRNA. To ensure that some RNA remains unspliced and is exported to the cytoplasm for incorporation into new virus particles the second intron contains a specific recognition sequence for a virally encoded protein called Rev. This RNA sequence is the Rev response element (RRE), for which a minimal functional sequence of 270 nucleotides has been identified (Huang *et al 1991).* Rev is a 13kD protein that specifically binds to RRE and activates the export of RRE-containing RNA (Malim *et al 1989b).* It appears that the HIV introns are recognised by the cellular splice commitment factors; but the splicing process is inefficient which leads to entrapment of the RNAs within the nucleus. The Rev/RRE interaction bypasses this entrapment and exports the RNA to the cytoplasm. If splicing is made more efficient then the RNA export becomes Rev independent (Chang and Sharp 1989; Hammarskjold *et al 1994).* If however gene expression is made more efficient then unspliced RNA can appear in the cytoplasm (e.g. D'Agostino *et al* 1992). In addition certain sequence elements referred to as cis inhibitory sequences (CRSs) might contribute to the Rev dependency of HIV by promoting nuclear entrapment and/or RNA instability (e.g. Cochrane *et al* 1991). There is therefore an interaction between splicing efficiency, expression efficiency and CRSs that may influence Rev dependency. The interaction is not necessarily predictable or definable and may vary in different cell types (as discussed by Cullen 1995). The mechanistic details of Rev-mediated export have not yet been established but cellular export proteins are involved (e.g. Fritz *et al* 1995).

One potential approach to achieving additional regulation of a retroviral vector for HIV gene therapy would therefore be to incorporate RRE into the vector rendering expression dependent upon Rev. Such an approach may be obvious for vectors specifically based upon HIV itself as these will of necessity contain the Rev/RRE system, unless removed. For example Naldini *et al* (1996) use a fragment of HIV genome encompassing the normal major splice donor sequence, RRE and the normal splice acceptor sequence in the normal configuration in their transducing vector pHR'. RRE is therefore contained within the HIV env intron. This fragment is presumed and is likely, but not proven, to render the expression of the genome responsive to Rev. In the vector described in this paper the coding sequence lies outside of the RRE containing intron.

A non-lentiviral retroviral vector incorporating the Rev/RRE system has been described by Lisziewicz (US patent publication PB92-139336) whereby the RRE element is inserted into a retroviral vector or into the intron of a foreign gene contained within that vector. The constructions outlined make no reference to the presence of the splice donor sequence within the MLV vector, nor take into account any requirement for inefficient splicing to achieve Rev function, and there is no description of precisely where to insert the RRE. The disclosure therefore suggests that in constructing a retroviral vector whose expression is dependent upon Rev the nature and location of the RRE is not material and the nature and location of additional introns or splice sequences is not material.

We now show that a TIN vector can be converted into a Rev responsive vector by the insertion of RRE to create TRIN (Tat and Rev inducible) vectors. However, contrary to the finding in Lisziewicz, we demonstrate that simply inserting RRE into a TIN vector does not achieve a strict dependency on Rev and that there is therefore no significant advantage in this construction with respect to reducing basal gene expression beyond that obtained with TIN vectors. Moreover, we show that the inclusion of an extended sequence derived from the HIV env region which contains RRE and the 3' splice acceptor sequence from env produces a new vector pTRAC for which the basal transcription levels are undetectable in the absence of Tat and Rev. We propose that this undetectable expression is due to the combination of nuclear retention of the RNA via recognition of a hybrid MLV/HIV intron and the fact that any RNA that is spliced rather than exported via the Rev system will have the reporter/therapeutic gene removed during splicing.

The invention therefore provides in one aspect a retroviral vector particle of claim 1 comprising a packagable RNA genome capable of being inserted into a target cell genome when in the form of a DNA provirus, said RNA genome carrying sequences which provide in the DNA provirus at least one selected gene capable of being expressed in the target cell and located within an intron in a transcription unit of the provirus, which transcription unit further comprises a polynucleotide response element responsive to a nucleus to cytoplasm transport factor, and wherein export of the transcribed gene from the nucleus to the cytoplasm is dependent on the nucleus to cytoplasm transport factor.

In another aspect, the invention provides a DNA construct encoding the packagable RNA genome for the retroviral vector particle described herein, under the control of a promoter. The selected gene or genes may be present in or absent from the DNA construct. If they are absent, the DNA construct has an insertion site e.g. a unique restriction enzyme site at which the selected gene or genes may be inserted, the site located within the intron such that it is flanked by a splice donor and a splice acceptor sequence.

In a further aspect, the invention provides a retroviral vector particle production system comprising a host cell transfected with a DNA construct as described herein, said system capable of producing retroviral vector particles as described herein. The host cell may be a packaging cell line or it may be a suitable host cell transfected with nucleic acid sequences, present e.g. on plasmids, encoding the structural elements of the retroviral particles. The transfected host cell is also referred to as a producer cell.

In yet another aspect, the invention provides a retroviral vector particle production system comprising a set of nucleic acid sequences encoding the components of a retroviral vector particle as described herein.

In a still further aspect, the invention provides the use of retroviral vector particles as described herein for gene therapy, and infected or transduced target cells resulting from such use.

The retroviral vector particle according to the invention therefore provides a means for inserting into a target cell a therapeutic gene whose expression in the target cell is dependent upon the presence of a factor which enables the transport of an RNA transcript containing the transcribed gene, into the cytoplasm.

The response element in the retroviral vector genome is chosen according to the specific conditions under which expression of the therapeutic gene is desired. If expression is to be HIV dependent, then a suitable response element is RRE or a functional equivalent which responds to Rev. A functional equivalent of RRE may be for example a portion of RRE or a mutated or otherwise manipulated version of RRE which retains the desired activity.

The transport factor may thus be HIV Rev, rendering expression of the therapeutic gene dependent upon the presence of HIV. The transport factor may alternatively be any factor, originating e.g. from other viruses or from host cells, which is analogous to Rev in that it enables transport to the cytoplasm via a specific interaction with the response element. Systems analogous to the Rev/RRE system are known, for example in other retroviruses. One such example is the rex/RxRE system in HTLV-1.

In order that RNA transcripts containing the transcribed therapeutic gene may be transported to the cytoplasm in the presence of the appropriate transport factor, the intron containing the therapeutic gene needs to be an inefficiently spliced intron. This may be achieved in a variety of ways. The preferred method described herein for achieving a Rev-dependent intron in an MLV-based vector is to use the MLV splice donor site and the HIV envelope gene 3' splice site. It is within the ability of those skilled in the art to devise other suitable splice site or intron sequence combinations for HIV Rev-dependency.

Preferably, the RRE or other response element is located within the intron, to ensure Rev- (or other transport factor) dependent expression. Conveniently, RRE and the 3' splice acceptor site may be provided in a sequence derived from the HIV env region.

Advantageously, the genome of the retroviral vector particle according to the invention will have its packaging site located within the intron containing the therapeutic gene. This means that RNA that is unspliced will be trapped in the nucleus and therefore unavailable for packaging, and RNA that is spliced will have the packaging site deleted and will also be unavailable for packaging. This feature contributes to the safety of the vector system, and is present in the preferred vector configuration described herein.

The retroviral vector particle may be MLV-based. MLV systems are so far the most widely used retroviral vector systems and have been used in human gene therapy applications. Other retroviruses may be used instead however, including other oncoretroviruses (the sub-group of retroviruses containing MLV), and lentiviruses (the sub-group of retroviruses containing HIV). Examples include ASLV, SNV and RSV all of which have been split into packaging and vector components for retroviral vector particle production systems. The retroviral vector particle according to the invention may be based on a genetically or otherwise (e.g. by specific choice of packaging cell system) altered version of a particular retrovirus.

That the vector particle according to the invention is "based on" a particular retrovirus means that the vector is derived from that particular retrovirus. The genome of the vector particle comprises components from that retrovirus as a backbone. The vector particle contains essential vector components compatible with the RNA genome, including reverse transcription and integration systems. Usually these will include gag and pol proteins derived from the particular retrovirus. Thus, the majority of the structural components of the vector particle will normally be derived from that retrovirus, although they may have been altered genetically or otherwise so as to provide desired useful properties. However, certain structural components and in particular the env proteins, may originate from a different virus. The vector host range and cell types infected or transduced can be altered by using different env genes in the vector particle production system to give the vector particle a different specificity.

Preferably, the retrovirus vector genome contains the minimum retroviral material necessary to function. This is important from a safety aspect; possible reconstruction of infectious virus particles must be avoided. In any case, the retroviral vector will be replication defective. Also, by avoiding expression of unwanted virus proteins in the target cell, undesirable immune responses are reduced. Gag-pol and env are therefore supplied in trans in the vector particle production system.

However the retrovirus vector genome clearly needs to contain the elements required for obtaining sufficiently high viral titres of vector particles from the producer cell, and for allowing it to integrate into the target cell genome. It will be evident that in order to function as a vector the retroviral vector particle according to the invention will need to have a reverse transcription system (compatible reverse transcription and primer binding sites) and an integration system (compatible integrase and integration sites) allowing conversion to the provirus and integration of the double-stranded DNA into the host cell genome. Additionally, the vector genome will need to contain a packaging signal.

In a vector particle according to the invention which is designed for use in gene therapy against a particular retrovirus, there is preferably also a control at the transcriptional level which requires the presence of that retrovirus in order to initiate or upregulate transcription of the therapeutic gene or genes. For anti-HIV gene therapy, the vector genome is preferably constructed such that in the DNA provirus the 5' LTR comprises HIV sequences sufficient to render the therapeutic gene Tat-inducible, and the therapeutic gene is located between the LTRs, yet the vector is based on a simple retroviral vector such as MLV. The therapeutic gene is under the transcriptional control of the promoter in the 5' LTR but not otherwise operably linked to any other promoter from the vector genome. This is the principle behind the TIN vectors discussed above.

In more detail, TIN vectors are constructed with the following points in mind. Tat acts on the TAR region of R, but also requires sequences in the U3 region to function properly. Certain sections of both U3 and R can be removed while still leaving an effective Tat-responsive element. Deletions in HIV-2 U3 result in promoters with lower basal levels of transcription that still remain responsive to Tat (Brady *et al* 1990). Thus, the transcriptional control is preferably provided by an HIV Tat-inducible promoter comprising the functional portions of both U3 and R from HIV. Alternatively certain U3 and/or R sequences from other retroviruses, which are responsive to the HIV Tat protein, may be used, for example U3 from RSV linked to R from HIV. The TIN vector provides a means of preserving the Tat response within the context of a simple retroviral vector e.g. one based on MLV such that MLV packaging systems may be used.

The selected gene located in the intron of the vector genome according to the invention is preferably a therapeutic gene, that is it encodes a gene product which is active against infection or disease. Where the vector particle is for use in anti-HIV gene therapy, the product of the therapeutic gene will have an appropriate activity for that purpose. Therapeutic genes may encode for example an anti-sense RNA, a ribozyme, a transdominant negative mutant of a target protein, a toxin, a conditional toxin, an antigen that induces antibodies or helper T-cells or cytotoxic T-cells, a single chain antibody or a tumour suppressor protein.

Where two or more genes are present in the same transcription unit, there may be an internal ribosome entry site (IRES) e.g. from picornaviral RNA, to allow both genes to be translated from a single transcript. Retroviruses incorporating IRES sequences have been constructed by others.

It will be evident that the term "gene" is used loosely here, and includes any nucleic acid coding for the desired polypeptide. Usually, the genes delivered by the vector particle according to the invention will be cDNAs.

A further gene may also be present outside the intron containing the therapeutic gene and under the control of a separate promoter. This further gene may encode for example a selectable marker, or a further therapeutic agent which may be among the therapeutic agents listed above. Expression of this gene may be constitutive; in the case of a selectable marker this may be useful for selecting successfully transfected packaging cells, or packaging cells which are producing particularly high titers of the retroviral vector. Alternatively or additionally, the selectable marker may be useful for selecting cells which have been successfully infected with the retroviral vector and have the provirus integrated into their own genome.

One way of performing gene therapy is to extract cells from a patient, infect the extracted cells with a retroviral vector and reintroduce the cells back into the patient. A selectable marker may be used to provide a means for enriching for infected or transduced cells or positively selecting for only those cells which have been infected or transduced, before reintroducing the cells into the patient. This procedure may increase the chances of success of the therapy. Selectable markers may be for instance drug resistance genes, metabolic enzyme genes, or any other selectable markers known in the art.

However, it will be evident that for many gene therapy applications of retroviral vectors, selection for expression of a marker gene may not be possible or necessary. Indeed expression of a selection marker, while convenient for *in vitro* studies, could be deleterious *in vivo* because of the inappropriate induction of cytotoxic T lymphocytes (CTLs) directed against the foreign marker protein. Also, it is possible that for *in vivo* applications, vectors without any internal promoters will be preferable. The presence of internal promoters can affect for example the transduction titres obtainable from a packaging cell line and the stability of the integrated vector.

The DNA construct according to the invention which encodes the packagable RNA genome preferably comprises a promoter originating from a source other than the first or second retrovirus. Particularly preferred are strong promoters such as the CMV promoter which give rise to a high level of expression of the vector RNA in the producer cell line.

The invention will now be further described with reference to the accompanying drawings in which:

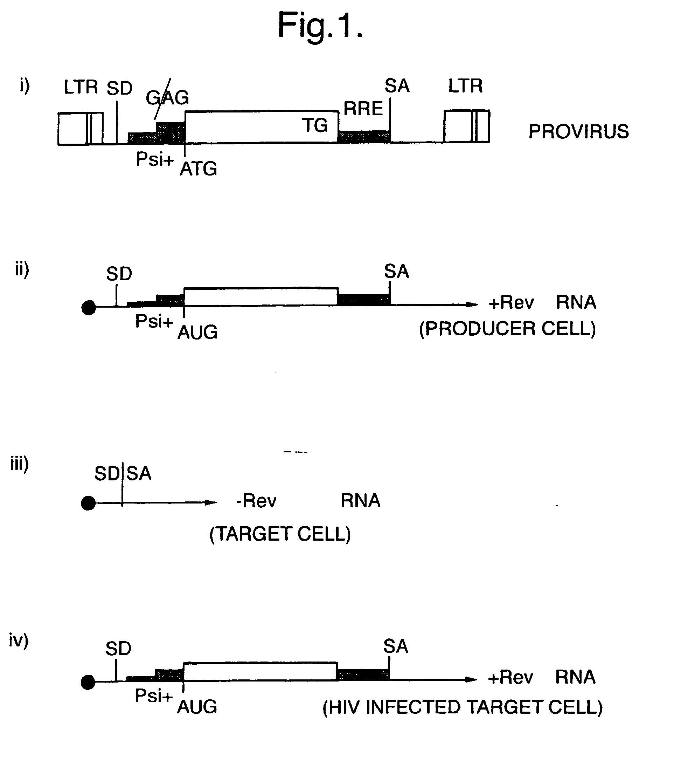

Figure 1 shows a schematic outline of constructs according to the invention. The vector genome in the producer cell is shown (i). In the producer cell Rev is provided either by cotransfection or in a stable cell line by procedures known to those ordinarily skilled. The resulting vector derived RNA (ii) is unspliced and is exported to the cytoplasm by the Rev protein. In the target cell the vector genome is as in (i) but the only RNA that appears in the cytoplasm has the structure shown in (iii) because Rev is absent. Upon HIV infection when Rev is produced by the infecting virus then the retroviral vector derived RNA that reaches the cytoplasm will have the structure shown in (iv) i.e. identical to that in the producer cell (ii);
Figure 2 shows vectors used in the construction of a universal TIN vector (pTIN511);
Figure 3 shows construction of an RRE splice acceptor cassette (RAC);
Figure 4 shows construction of pTRAC;
Figure 5 shows construction of pTRAC-TG;
Figure 6 shows construction of pTRIN-TG; and
Figure 7 shows the prototype TIN vector pTIN414.

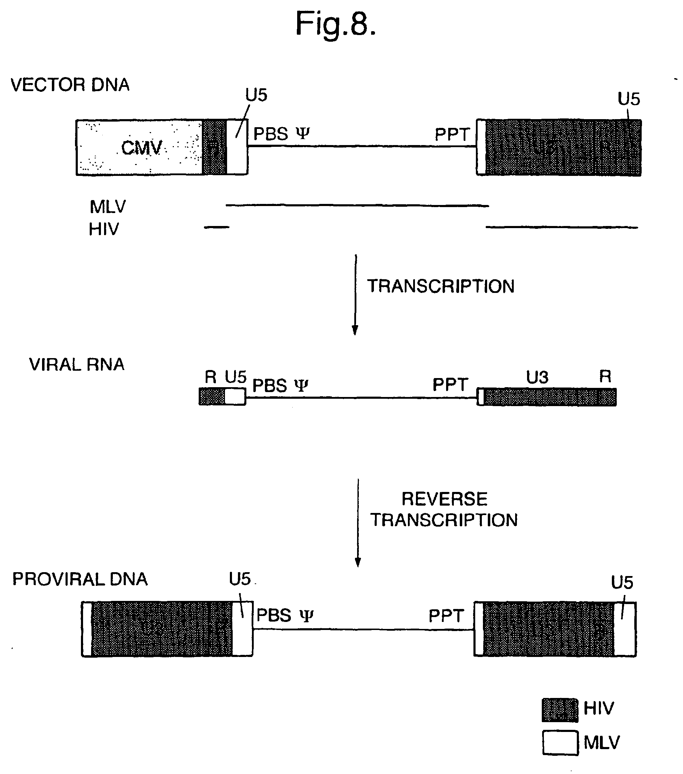

Figure 8 shows the principle of TIN vectors. The specific example of an MLV-based Tat-inducible vector is given. The packagable RNA genome comprises from the 5'end at least the functional part of the R region of HIV, all or a functional part of the MLV U5 region, a functional MLV primer binding site for first strand reverse transcription, a functional MLV packaging site, an insertion site for insertion of one or more therapeutic genes (into the DNA copy), a functional MLV primer binding site for second strand reverse transcription, a short (e.g. 10-100 nucleotides) sequence recognised by the MLV integration system, all or a substantial part of the HIV U3 region, and an R region corresponding to the R region at the 5'end. Vector and proviral DNA are also shown.

Preparation of the constructs is described in detail in the Examples which follow.

This is the first description of the incorporation of an extended HIV-1 RRE element combined with an HIV-1 splice acceptor site into an MLV vector. It is the first description of a retroviral vector where the therapeutic gene is contained within an intron. The invention is particularly useful when combined with a TIN vector. That vector could be a single transcription unit vector or could contain a second transcription unit as described for pTIN501. The retroviral vector could be based on any retrovirus including lenti-viruses. In the case of lentiviral vectors a vector has been described that contains the HIV-1 RRE and the major splice site but the therapeutic gene is not included within an intron and the Rev/RRE system is not used to manipulate the expression of the therapeutic gene (Naldini *et al* 1996). The components of the intron in the present invention are preferably derived from MLV and HIV.

### COMPONENTS OF MLV BASED TAT-INDUCIBLE (TIN) VECTORS

We have constructed a series of vectors that are packagable by standard MLV components, can be reverse transcribed and integrated by the MLV machinery following infection or transduction of a cell, and will allow Tat-inducible expression of a therapeutic gene from the transduced vector. The basic components of such a vector are as follows (Figure 8):
**1. Tat-inducibility.** The Tat-inducibility of the HIV LTR promoter is a property of sequences in the U3 region of the promoter and also the TAR element in R (Berkhout and Jeang, 1992). In addition, some other promoters, including the U3 regions from HIV-2 and RSV can substitute for the HIV-1 U3 regions to allow Tat transactivation (Liu *et al.* 1994). In order for the U3 element to appear in the 5' LTR following reverse transcription, it must be present in the 3' LTR of the viral RNA. The vector therefore contains the HIV U3 and R sequences at the 5'LTR.
**2. Reverse transcription**. MLV RT initiates reverse transcription at the primer binding site (PBS). This initial (-) strand synthesis extends into U5 and R sequences, forming the first 'strong stop' DNA strand. The RNAseH moiety of RT then degrades the RNA in this hybrid, allowing the exposed DNA to hybridise with the homologous R region in the 3' LTR of the provirus. The homology between the 5' and 3' R regions enables the polymerase to switch strands and continue synthesis along the (-) strand from the 3'LTR. (+) strand DNA synthesis is primed by the selective retention of an RNA fragment at the polypurine tract after RNAse degradation of the genomic RNA strand (reviewed in Katz and Skalka, 1994). The minimum requirements for MLV pol directed reverse transcription contained in the vector are therefore the PBS to initiate (-) strand DNA synthesis, the PPT to initiate (+) strand DNA synthesis and identical 5' and 3' R sequences to allow the first template switch. The requirement for identical R sequences is met by having HIV R sequences in both 5' and 3' LTRs. In addition, as there is evidence to suggest that secondary structures in the 5' U5 region are also important for the initiation of reverse transcription (Cobrink *et al.* 1991), we have kept the MLV U5 sequences in the 5'LTR. The 3' U5 sequences do not appear in the genomic RNA transcript; however, to ensure correct termination at the 3' R/U5 border during genomic transcription, we will use the HIV U5 region in the 3' LTR.
**3. Integration.** The termini of the reverse-transcribed molecule contain short, sometimes imperfect, inverted repeats of 2-23 bp, which the retroviral integrase recognises (reviewed in Katz and Skalka, 1994). For MLV, it has been demonstrated that only 9 bases at the end of a linear model substrate are sufficient for almost wild-type levels of integration in an in vitro integration assay system (Bushman and Craigie, 1990). These sequences are derived from the ends of the 3'U3 region and the 5'U5 region in the vector. This requirement is met by the vector containing 36 bases of MLV sequence at the 5' end of the 3' U3 and the whole of the MLV 5' U5 region.
**4. Packaging components**. Efficient packaging of a vector genome into a retroviral particle is dependent on a number of cis-acting sequences (reviewed in Linial and Miller, 1990). The most important sequence is the packaging signal, a highly structured region of RNA₋at the 5' region of the genome. In addition, other regions of the genome have been found to increase the efficiency of packaging, including sequences in gag p15. This region is included in standard MLV retroviral vectors, such as LXSN (Miller and Rosman, 1989) and is also preserved in our constructs.
**5. High titer retrovirus stocks following transient transfection**. We have recently devised a system for the rapid production of high titer retroviral vectors (10⁷/ml) by transient transfection (Soneoka *et al.* 1995). A key feature of this system is that the powerful CMV promoter drives high level expression of the vector RNA in the producer cell line and is positioned so that the transcription start site of the vector RNA is exactly the same as the normal LTR-directed start site. To achieve this, we have placed the CMV promoter up to its transcriptional start site adjacent to the start of the HIV R region in the 5' LTR. This principle could be applied to other heterologous promoters, as long as the integrity of the retroviral transcription unit is maintained.

This system has been described for hybrid MLV-HIV vectors, but the same principle can be applied to other combinations of retroviruses, where one retrovirus is donating the cis-acting sequences required by its own packaging components provided in trans (e.g. SNV, RSV. ASLV etc.) and the other retrovirus is used because of the property of conditional expression of its LTR promoter. Further examples of such retroviral promoters are the HTLV-1 promoter (dependent on Tax protein) and the steroid-hormone inducibile MMTV LTR (reviewed in Majors, 1990). The use of HTLV-1 LTR, for example, to direct expression of a suicide gene could find applications as a treatment for adult T-cell leukemia.

### ADVANTAGES OF THE VECTORS DESCRIBED HEREIN

1. Reduced basal levels of expression of therapeutic genes leading to reduced toxicity and immune recognition of target cells.
2. Stricter dependency upon HIV infection to activate gene expression
3. Reduced availability of RNA carrying the packaging site leading to lower risk of inadvertent packaging of the RNA in the target cell.

### EXAMPLES

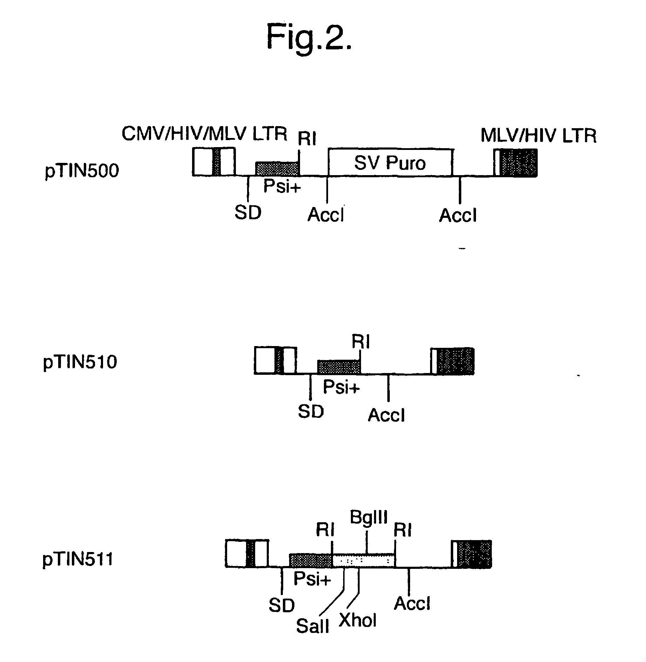

### I. Construction of a universal TIN vector (pTIN511) (Figure 2)

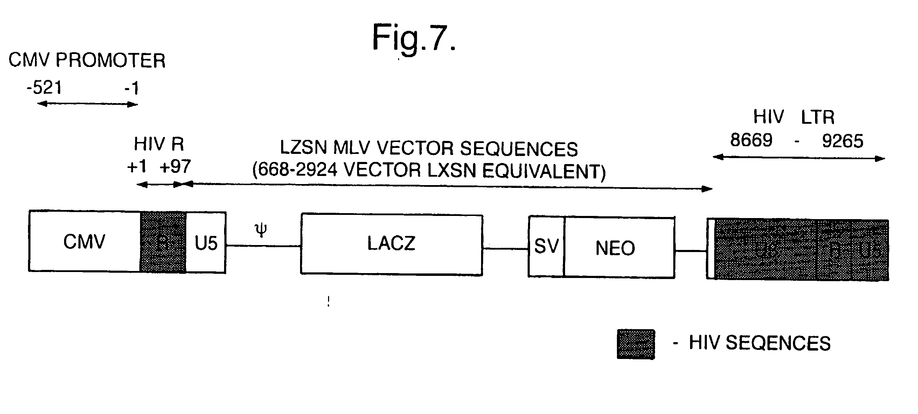

The starting molecule is pTIN500 (described in PCT/GB96/01230). This was derived from pTIN414 described in PCT/GB96/01230 (and described herein in detail below and illustrated in Figure 7). PTin414 is the TIN vector equivalent of retroviral vector PHIT111 (Soneoka *et al* 1995), a derivative of LZSN (Adam *et al* 1991). Subsequent TIN vectors were derived from pTIN414 by replacement of internal sequences between unique Spel and Nhel sites. The Spel site is located within the non-translated gag coding region upstream of the lacZ gene and the Nhe I site is in the 3' U3 region at the junction of the MLV and HIV-1 sequences. Plasmid pTIN500 contains the Spel - Nhel internal fragment from pBABEpuro. The SV-Puro cassette is deleted by digestion with Accl and religation. This produces pTIN510. A poly-linker is inserted into the unique EcoRl site in pT1N510.

### Polylinker sequence (SEQ ID NO: 1)

EcoRI SalI XhoI BglII EcoRI
5'AATTCGTCGACCTCGAGATCCG;
GCAGCTGGAGCTCTAGGCAATT 5'

This creates pTIN511 which has unique Sall, Xhol and BgIII sites for the insertion of additional sequences.

The removal of the SV-Puro cassette is not critical for the current invention but serves to simplify the structure of the vector. There may be situations, obvious to one skilled in the area, when the retention of this or any additional cassette might be desirable.

### Detailed construction of TIN414.

The co-ordinates of the sequences derived from the CMV promoter, the MLV vector LZSN (Adam *et al.* 1991) and the HIV-1 proviral clone WI3 (Kim *et al*. 1989) are indicated. The molecule was created using standard recombinant DNA techniques and in addition, recombinant PCR to create exact junctions between the different parts of the molecule (Higuchi 1990). Specifically

### (i) Construction of the 5' CMV driven LTR

Plasmid pPE611 (Braddock *et al*. 1989) contains the human CMV promoter (from -521 to +1) joined exactly to the start of the HIV-1 R region (co-ordinates +1 to +80). An Xbal - BamHl fragment from this plasmid was ligated into the cloning vector pBluescript (Stratagene) to give plasmid pRV404. A PCR amplification was performed using plasmid pLNSX as the template, using primers 5'-gcgagctagcttcgaatcgtggtctcgctgttccttgg-3' and 5'ggccgctagcgttcagaactcgtcagttccaccac-3'. The PCR product so generated was digested with Nhel and ligated into pRV404 at its Nhel site to give plasmid pRV405. Two oligonucleotides of sequence 5'-ttaagcctcaataaagcttgccttgagtgcttcatc-3' and 5'-cggatgaagcactcaaggcaagctttattgaggc-3' were annealed together to create a short duplex containing single stranded regions at either end corresponding to the overhangs present on AfIII and BstBI restriction fragments. This molecule was ligated into plasmid pRV405 cut with AfIII and BstBI, to give plasmid pRV406.

### (ii) Construction of the 3' LTR

A Hindlll - Xbal fragment from pLNSX was ligated into the cloning vector pSP72 to give plasmid pRV400. Plasmid pBX+ contains a BamHI - Xbal fragment from WI3 containing the whole 3' LTR from the HIV-1 genome. PCR amplification was performed on pBX+ using primers 5'-ccgcgctagcgatatccttgatctgtggatctaccac-3' and 5'gcgagggtaccgtcgactgctagagattttccacactgac-3'. The PCR product was digested with KpnI and Nhel and ligated into plasmid pRV400 digested with Kpnl and Nhel, to give plasmid pRV401. The Clal - Kpnl fragment of pRV401 was ligated into pBluescript digested with Clal and Kpnl, to give plasmid pRV408.

### (iii) Addition of internal sequences.

A Sacll - Spel fragment from pRV406 was ligated into plasmid pRV408 to create plasmid pRV412. The Spel -Nhel fragment from pRV412 was replaced with the Spel - Nhel fragment from LZSN to give vector pTIN414 (Figure 7).

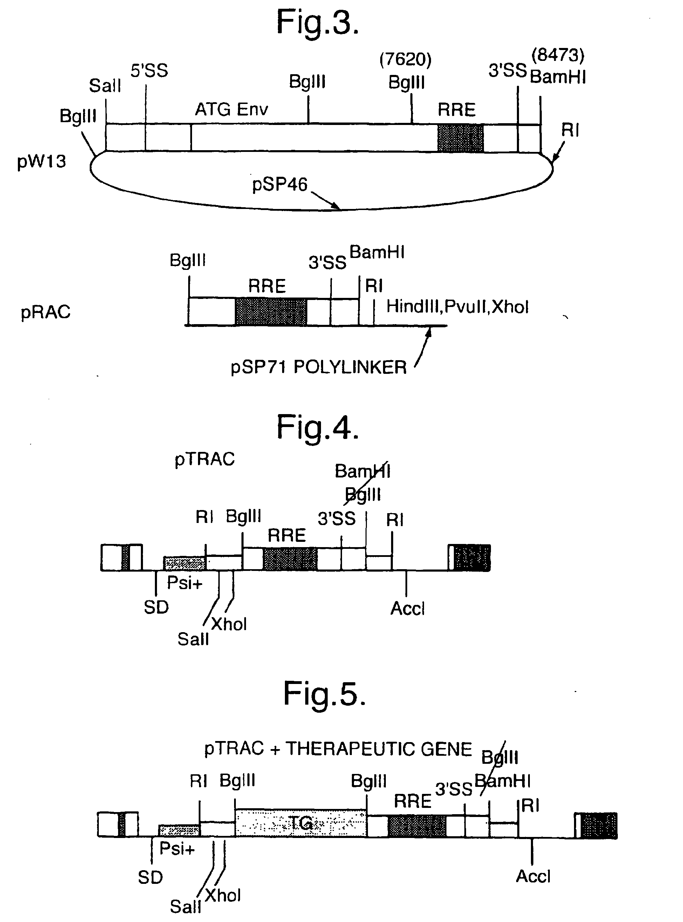

### 2. Construction of the RRE splice acceptor cassette (RAC) Figure 3.

The starting molecule is pWI3 (Kim *et al* 1989). This contains a full length proviral clone of isolate HIV-1 IIIb clone HXB2 (Ratner *et al* 1985; Genbank accession no.K03455). Coordinates refer to nucleotide positions in the proviral DNA starting with the first nucleotide of the 5' LTR as 1 and are given in brackets following the restriction site. To facilitate cloning steps a subclone is created that contains only the envelope region of HIV-1. To achieve this a Sall (5785) to BamHI (8473) fragment from WI3 is inserted into the polylinker of the cloning vector pSP46 (Promega) to produce plasmid pPE531. The RRE splice acceptor region (RAC) is further subcloned as a BgIII (7620) to EcoRI (site in the pSP46 polylinker) into the polylinker site of pSP71 to create pRAC.

### 3. Construction of pTRAC (Figure 4)

The BgIII(7620) to BamHI (8473) fragment is inserted into the unique BgIII site in pTIN511. In the correct orientation the upstream BgIII site is preserved and the downstream site is a hybrid BgIII/BamHI site which is non-functional for either enzyme pTRAC therefore retains three unique cloning sites upstream of the RAC .

### 4. Construction of pTRAC-TG (Figure 5)

A coding sequence of choice is inserted into one of the unique restriction sites in pTRAC. This sequence is a therapeutic gene or a reporter gene. The sequence is prepared with appropriate restriction at the termini and has an ATG codon for translation initiation. In the present example the RevM10 sequence is amplified from plasmid pM10 (Malim *et al* 1989a) using PCR primers incorporating flanking BgIII (upstream) and BamHI (downstream sites).

### Primer sequences:

GGCAGATCTATGGCAGGAAGAAGCGG - 3' (SEQ ID NO: 2)
GGCGGATCCTTCTTTAGTTCCTGACTCC - 3' (SEQ ID NO: 3)

The amplified product is digested with BgII and BamHI and the product is ligated into the unique BgIII site of pTRAC. The vector genome is renamed according to the therapeutic gene in this example pTRAC-TG becomes pTRAC-RevM10.

Insertion of the gene in the correct orientation preserves the upstream BgIII site but the downstream site is destroyed by the formation of a BgIII/BamHI hybrid site.

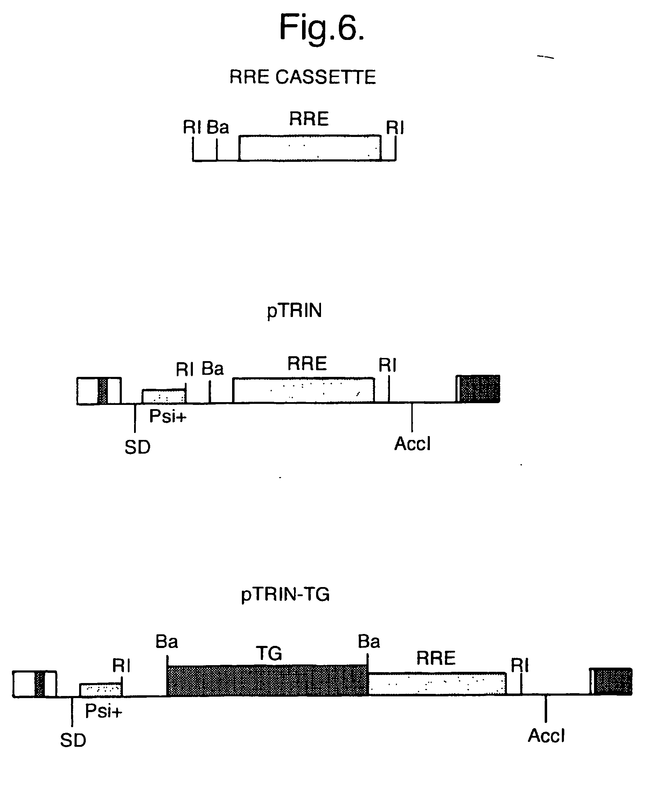

### 5. Construction of pTRIN and pTRIN-TG (Figure 6)

To construct an RRE cassette, a 359 base pair fragment encompassing the minimal fully functional RRE is amplified from pRAC using primers that locate with respect to the HIV proviral sequence coordinates at nucleotide 7705 and 8067 (or from pPE351 sequences from 7707 to 8066). The upstream primer adds an EcoRI site followed by a BamHI site and the downstream primer adds an EcoRI site.
Primer 1 (SEQ ID NO: 4) (lower case is HIV-1 sequence 7705 to 7725) 5'CCGCGAATTCGGATCCaggagtagcacccaccaaggc
Primer 2 (SEQ ID NO: 5) (lower case is HIV-1 sequence from 8067 to 8047) 5'CCGCGAATTctccaactagcattccaaggc

The amplified product is digested with EoRI and is ligated into the EcoRI site of pTIN510 to produce pTRIN. This now has a unique BamHI site for the insertion of any additional sequences.

A therapeutic gene or reporter gene is inserted into the unique BamHI site of pTRIN. In the present example the RevM10 cassette described above is inserted. The vector is renamed according to the therapeutic gene in this example pTRIN-TG becomes pTRIN-RevM10.

### 6. Analysis of pTRAC-TG and pTRIN-TG

Retroviral vector stocks are produced either by transient transfection of 293T cells according to methods of Soneoka *et al* 1995 or by the creation of producer cell lines by standard methods e.g. (Cosset *et al* 1995). MLV packaging components are provided in trans on two plasmid components - a gag-pol expression plasmid (pHIT60, an MLV gag-pol expression plasmid described in Soneoka *et al* 1995) and an amphotropic envelope expression plasmid (pHIT456 derived from plasmid SV-A-MLV-env described in Page *et al* 1990 and essentially the same as the ecotropic expression construct pHIT123 described in Soneoka *et al* 1995)*.* Rev is provided in trans from a CMV rev expression plasmid. The following vector genomes are used:-
a) standard MLV vector (pHITIII; Soneoka *et al* 1995)
b) pTRIN-RevM10
c) pTRAC-RevM10
d) pTRIN-lacZ
e) pTRAC-lacZ
f) pTIN414 (as described in PCT/GB96/01230) and shown in Figure 7.
   The following additional plasmids are used in the analysis:-
g) pTAT (A CMV Tat expression plasmid as described by Braddock *et al* 1989)
h) pHCMVsrev (A Rev expression plasmid as described by Benko *et al* 1990).

Virus stocks are used to infect HeLa-CD4 cells and human U937 cells. In some cases repeat transfections are done as described by Cannon *et al* (1996). In this procedure the target cells are exposed to fresh retroviral vector stocks every 48 hours. Forty-eight hours after the final transduction the transduced cell populations are infected with HIV-1. After 48 and 72 hours cells are harvested and protein extracts are prepared by standard procedures and assayed for the expression of the marker or therapeutic gene. In addition viral spread through the culture is assessed by determining reverse transcriptase activity in culture supernatants every three days.

### REFERENCES

Adam, M.A., N. Ramesh; A.D. Miller and W.A. Osborne (1991) J. Virol. 65: 4985-4990.
Arya, S K., C. Guo, S.F. Josephs and F. Wong-Staal. (1985). Science 229:69-73.
Baltimore, D. (1988). Nature 335:395-396.
Benko et al (1990) The New Biologist, 2:1116.
Berkhout, B. and K.-T. Jeang (1992). J. Virol. 66:139-149.
Braddock et al (1989) Cell, 58: 269.
Brady, H., C.G. Miles, D.J. Pennington and E.A. Dzierzak. (1994). PNAS 91:365
Bushman, F.D. and R. Craigie. (1990). J. Virol. 64:5645-5648.
Caruso, M. and D. Klatzman. (1992). PNAS 89:182-186.
Chang & Sharp (1989) Cell 59: 789.
Cobrink, D., A. Aiyar, Z. Ge, M. Katzman, H. Huang and J. Leis. (1991). J. Virol 65:3864.
Cochrane et al (1991) J. Virol. 65:5305.
Cosset et al (1995) J. Virol. 69:7430.
Cullen, B.R. (1995) AIDS vol 9 Suppl A: 19-32.
D'Agostino et al (1992) Mol. Cell. Biol. 12:1375.
**Felber** ***et al**.*
Fritz et al (1995) Nature 376: 530.
Hammarskjold et al (1994) J. Virol. 68: 951.
Higuchi, R. (1990) Recombinant PCR, p.177-183. In M.A. Innis, D.H. Gelfand, J.J.Snisky and T.J. White (Eds), PCR Protocols, Academic Press, San Diego.
Huang et a/ (1991) J. Virol. 65: 2131.
Katz, R.A. and A.M. Skalka. (1994). Annu. Rev. Biochem. 63:133-173. **Linial, M.L**. and A.D. Miller. (1990). Ed. R. Swanstrom and P.K. Vogt. Springer-Verlag.
Liu, J., C. Woffendin, Z. Yang and G.J. Nabel. (1994). Gene Therapy 1:32-37.
**Majors, J**. (1990). Ed. R. Swanstrom and P.K. Vogt. Springer-Verlag.
Malim, M.H., S. Bohnlein, J. Hauber and B.R. Cullen (1989a). Cell 58:205-214.
Malim, M.H. et al (1989b) Nature 338: 254.
Miller, A.D. and G.J. Rosman. (1989). Biotechniques 7:980-990.
Nabel & Baltimore (1987) Nature 326: 711
Naldini et al (1996) Science 272:263
Page K.A., N.R. Landau and D.R. Littman (1990) J. Virol. 64: 5270-5276.
Ratner et al (1995) Nature, 313: 277.
Soneoka, Y., P.M. Cannon, E.E. Ramsdale. J.S. Griffiths, G. Romano, S.M. Kingsman and A.J. Kingsman. (1995). Nucl. Acids Res. 23: 628-633.
Valerie et al (1988). Nature 333: 78-81.
Yu, S.-F., T. von Ruden, P.W. Kantoff, C. Garber, M. Seiberg, U. Ruther, W.F. Anderson, E F. Wagner and E. Gilboa. (1986). PNAS 83:3194-3198.
Yu; M., E. Poeschla and F. Wong-Staal (1994) Gene Therapy 1: 13-26.

## Claims

1. A retroviral vector particle based on a first retrovirus which is not HIV, comprising a packagable RNA genome capable of being inserted into a target cell genome when in the form of a DNA provirus, said RNA genome carrying sequences which provide in the DNA provirus (i) a splice donor site from the first retrovirus, and ii) at least one selected gene capable of being expressed in the target cell and located within an intron in a transcription unit of the provirus, which transcription unit further comprises the 3' splice acceptor site from HIV and the REV response element (RRE), rendering expression of the selected gene responsive to HIV REV.

2. The retroviral vector particle according to claim 1, wherein a selected gene is a therapeutic gene.

3. The retroviral vector particle according to claim 1 or 2, wherein the first retrovirus is an oncoretrovirus.

4. The retroviral vector particle according to any preceding claim wherein the first retrovirus is murine leukaemia virus (MLV).

5. The retroviral vector particle according to any one of claims 1 to 4 wherein the 5' long terminal repeat (LTR) of the provirus comprises HIV U3 and R regions or functional portions thereof having Tat inducible promoter activity, in place of the 5' LTR promoter function of the retrovirus on which the vector particle is based.

6. The retroviral vector particle according to any one of claims 1 to 5, wherein the packaging signal is contained within the intron in which the selected gene is located.

7. The DNA construct encoding the packagable RNA genome for the retroviral-vector particle according to any one of the claims 1 to 6, operably linked to a promoter.

8. The DNA construct according to claim 7, wherein the promoter is the CMV promoter.

9. The DNA construct according to claim 7 or claim 8, wherein the selected gene is absent and the construct has an insertion site within the intron at which the selected gene or genes may be inserted.

10. A retroviral vector particle production system comprising a host cell transfected with the DNA construct according to any one of claims 7 to 9, said system capable of producing retroviral vector particles according to any one of claims 1 to 6.

11. A retroviral vector particle production system comprising a set of nucleic acid sequences encoding the components of a retroviral vector particle according to any one of claims 1 to 6.

12. The use of a retroviral vector according to any one of claims 1 to 6 in the preparation of a medicament for gene therapy for infection or transduction of a target cell.

13. Target cells resulting from the method according to claim 10.

## Patentansprüche

1. Retrovirales Vektorpartikel, basierend auf einem ersten Retrovirus, welcher nicht HIV ist, das ein verpackbares RNA-Genom, welches in ein Zielzellgenom eingebracht werden kann, wenn es in der Form eines DNA-Provirus vorliegt, umfaßt, wobei das RNA-Genom Sequenzen trägt, die in dem DNA-Provirus (i) eine Splice-Donor-Stelle von dem ersten Retrovirus und (ii) wenigstens ein ausgewähltes Gen, welches in der Zielzelle exprimiert werden kann und innerhalb eines Introns in einer Transkriptionseinheit des Provirus angeordnet ist, wobei die Transkriptionseinheit weiterhin die 3'-Splice-Akzeptor-Stelle von HIV und das REV-Antwortelement (RRE) umfaßt, bereitstellen, wodurch das ausgewählte Gen in Reaktion auf HIV-REV exprimiert wird.

2. Retrovirales Vektorpartikel nach Anspruch 1, wobei ein ausgewähltes Gen ein therapeutisches Gen ist.

3. Retrovirales Vektorpartikel nach Anspruch 1 oder 2, wobei der erste Retrovirus ein Onkoretrovirus ist.

4. Retrovirales Vektorpartikel nach einem der vorangegangenen Ansprüche, wobei der erste Retrovirus Maus-Leukämievirus (MLV) ist.

5. Retrovirales Vektorpartikel nach einem der Ansprüche 1 bis 4, wobei die 5'-lange terminale Wiederholung (LTR) des Provirus HIV-U3- und -R-Regionen oder funktionale Teile davon mit Tat-induzierbarer Promotoraktivität anstelle der 5'-LTR-Promotorfunktion des Retrovirus, auf welchem das Vektorpartikel basiert, umfaßt.

6. Retrovirales Vektorpartikel nach einem der Ansprüche 1 bis 5, wobei das Verpackungssignal in dem Intron, in welchem das ausgewählte Gen liegt, enthalten ist.

7. DNA-Konstrukt, welches das verpackbare RNA-Genom für das retrovirale Vektorpartikel nach einem der Ansprüche 1 bis 6 codiert, in funktionsfähiger Verknüpfung mit einem Promotor.

8. DNA-Konstrukt nach Anspruch 7, wobei der Promotor der CMV-Promotor ist.

9. DNA-Konstrukt nach Anspruch 7 oder Anspruch 8, wobei das ausgewählte Gen fehlt und das Konstrukt eine Insertionsstelle innerhalb des Introns aufweist, an welcher das ausgewählte Gen oder die Gene eingebracht werden kann bzw. können.

10. System zur Herstellung eines retroviralen Vektorpartikels, welches eine Wirtszelle, transfiziert mit dem DNA-Konstrukt nach einem der Ansprüche 7 bis 9, umfaßt, wobei das System retrovirale Vektorpartikel nach einem der Ansprüche 1 bis 6 produzieren kann.

11. System zur Herstellung eines retroviralen Vektorpartikels, welches einen Satz von Nukleinsäuresequenzen, die die Komponenten eines retroviralen Vektorpartikels nach einem der Ansprüche 1 bis 6 codieren, umfaßt.

12. Verwendung eines retroviralen Vektors nach einem der Ansprüche 1 bis 6 bei der Herstellung eines Medikaments für die Gentherapie für die Infektion oder Transduktion einer Zielzelle.

13. Zielzellen, die aus dem Verfahren nach Anspruch 10 resultieren.

## Revendications

1. Particule de vecteur rétroviral à base d'un premier rétrovirus qui n'est pas le VIH, comprenant un génome à ARN encapsidable capable d'être inséré dans le génome d'une cellule cible lorsqu'il est sous forme d'un provirus à ADN, ledit génome à ARN portant des séquences qui fournissent dans le provirus à ADN (i) un site donneur d'épissage provenant du premier rétrovirus et (ii) au moins un gène choisi capable d'être exprimé dans la cellule cible et situé à l'intérieur d'un intron dans une unité de transcription du provirus, unité de transcription qui comprend en outre le site accepteur d'épissage 3' du VIH et l'élément de réponse à REV (RRE), rendant l'expression du gène choisi sensible au REV du VIH.

2. Particule de vecteur rétroviral suivant la revendication 1, dans laquelle un gène choisi est un gène thérapeutique.

3. Particule de vecteur rétroviral suivant la revendication 1 ou 2, dans laquelle le premier rétrovirus est un oncorétrovirus.

4. Particule de vecteur rétroviral suivant l'une quelconque des revendications précédentes, dans laquelle le premier rétrovirus est le virus de la leucémie murine (MLV).

5. Particule de vecteur rétroviral suivant l'une quelconque des revendications 1 à 4, dans laquelle la longue répétition terminale (LTR) 5' du provirus comprend les régions U3 et R du VIH ou leurs portions fonctionnelles ayant une activité de promoteur inductible Tat à la place de la fonction de promoteur LTR 5' du rétrovirus servant de base de la particule de vecteur.

6. Particule de vecteur rétroviral suivant l'une quelconque des revendications 1 à 5, dans laquelle le signal d'encapsidation est présent à l'intérieur de l'intron dans lequel le gène choisi est situé.

7. Produit d'assemblage d'ADN codant pour le génome à ARN encapsidable pour la particule de vecteur rétroviral suivant l'une quelconque des revendications 1 à 6, lié de manière fonctionnelle à un promoteur.

8. Produit d'assemblage d'ADN suivant la revendication 7, dans lequel le promoteur est le promoteur du CMV.

9. Produit d'assemblage d'ADN suivant la revendication 7 ou la revendication 8, dans lequel le gène choisi est absent et le produit d'assemblage possède un site d'insertion à l'intérieur de l'intron au niveau duquel peuvent être insérés le ou les gènes choisis.

10. Système de production d'une particule de vecteur rétroviral, comprenant une cellule hôte transfectée avec le produit d'assemblage d'ADN suivant l'une quelconque des revendications 7 à 9, ledit système étant capable de produire des particules de vecteur rétroviral suivant l'une quelconque des revendications 1 à 6.

11. Système de production d'une particule de vecteur rétroviral, comprenant une série de séquences d'acide nucléique codant pour les constituants d'une particule de vecteur rétroviral suivant l'une quelconque des revendications 1 à 6.

12. Utilisation d'un vecteur rétroviral suivant l'une quelconque des revendications 1 à 6 dans la préparation d'un médicament destiné à la thérapie génique pour l'infection ou la transduction d'une cellule cible.

13. Cellules cibles résultant du procédé suivant la revendication 10.
